# EUROPEAN PATENT SPECIFICATION

(11) **EP 3 270 925 B1**
(45) Date of publication and mention of the grant of the patent: **24.11.2021**
(21) Application number: 17720876.6
(22) Date of filing: 31.03.2017
(51) Int. Cl.: A61K 31/497, A61K 31/43, A61P 31/04

(54) **ANTIBACTERIAL COMPOSITIONS**
ANTIBAKTERIELLE ZUSAMMENSETZUNGEN
COMPOSITIONS ANTIBACTÉRIENNES

(30) Priority: 31.03.2016 IN 201621011250
(43) Date of publication of application: 24.01.2018
(73) Proprietor: Wockhardt Limited, Aurangabad 431006 (IN)
(72) Inventor: NANDANWAR, Manohar Baburao, Aurangabad - 431005, Maharashtra (IN); KANSAGARA, Atul, Aurangabad - 431001, Maharashtra (IN); NAGORI, Rajendra Nandlal, Aurangabad - 431210 (IN); PATEL, Muftedar Ahmed Iftekhar, Aurangabad - 431001, Maharashtra (IN); YEOLE, Ravindra Dattatraya, Aurangabad - 431003, Maharashtra (IN); CHAUHAN, Bhaskar, Chandausi, District : Sambhal - 202412, Uttar Pradesh (IN); DEO, Keshav, VADODARA - 390021, Gujarat (IN); PATEL, Mahesh Vithalbhai, Aurangabad 431003, Maharashtra (IN)
(74) Representative: Nederlandsch Octrooibureau
(86) International application number: PCT/IB2017/051871
(87) International publication number: WO 2017/168393

(56) References cited:
- WO-A1-2012/164358
- WO-A2-2007/129176
- CN-B- 102 743 388
- FUBARA J O ET AL: "Influence of pH, temperature and buffers on cefepime degradation kinetics and stability predictions in aqueous solutions", JOURNAL OF PHARMACEUTICAL SCIENCES, vol. 87, no. 12, 1 December 1998 (1998-12-01), pages 1572-1576, XP002628079, AMERICAN PHARMACEUTICAL ASSOCIATION, WASHINGTON, US ISSN: 0022-3549, DOI: 10.1021/JS980170Y [retrieved on 2000-06-08]
- GHAFUR ET AL: "A new beta-lactam/beta-lactamase inhibitor combinationClinical profile of patients treated with cefepime/tazobactam.", J MICROBIOL. INFECT. DIS., vol. 2, no. 3, September 2012 (2012-09), pages 79-86, XP002771409, DOI: 10.5799/ahinjs.02.2012.03.0049

## Description

### FIELD OF THE INVENTION

The invention relates to antibacterial compositions and their use in methods for treatment, control or prevention of bacterial infections.

### BACKGROUND OF THE INVENTION

Infections caused by bacteria continue to remain an area of serious concern worldwide. One of the key challenges in the treatment, control or prevention of bacterial infections is the ability of bacteria to develop resistance to one or more antibacterial agents over time. Representative examples of such bacteria that have developed resistance to typical antibacterial agents include: Penicillin-resistant *Streptococcus pneumoniae,* Vancomycin-resistant *Enterococci,* and Methicillin-resistant *Staphylococcus aureus.* The problem of emerging drug-resistance in bacteria is often tackled by switching over to newer antibacterial agents. However, development of new antibacterial agents can be expensive and may not be always a permanent solution as bacteria often develop resistance to the newer antibacterial agents in due course. In general, bacteria are often efficient in developing resistance to antibacterial agents because of their ability to multiply very rapidly and pass on the resistance genes as they replicate. Bacteria develop resistance to existing antibacterial agents through various mechanisms including production of beta lactamases, mutations in the Penicillin-binding proteins (PBPs), development of efflux pumps, and decreased expression of outer membrane proteins or porins. For example, in response to the continued exposure to a variety of beta-lactam antibacterial agents, bacteria have developed several types of beta lactamases that are capable of hydrolyzing antibacterial agents belonging to penicillins, cephalosporins, monobactams and even carbapenems.

There is an urgent need for development of newer ways to treat bacterial infections, and in particular, infections caused by bacteria that have acquired resistance to one or more of the existing antibacterial agents. A composition comprising at least one antibacterial agent and tazobactam was disclosed in PCT International Patent Application No. PCT/IB2011/053398. For example, a composition comprising cefepime and tazobactam exhibited a synergistic antibacterial effect against a wide variety of bacteria. However, a combination of cefepime and tazobactam when administered intravenously caused inflammation of veins (the effect also known as phlebitis). The inventors have now surprisingly discovered that it is possible to use a composition comprising cefepime and tazobactam without causing phlebitis, if a specific amount of arginine or a pharmaceutically acceptable salt thereof is added to the composition before administration, and also by carefully maintaining pH of the composition within a certain range during the administration.

### SUMMARY OF THE INVENTION

Accordingly, there is provided a pharmaceutical composition suitable for parenteral administration, said composition comprising: (a) 2 gram of cefepime or a pharmaceutically acceptable salt thereof, (b) 2 gram of tazobactam or a pharmaceutically acceptable salt thereof, and (c) arginine or a pharmaceutically acceptable salt thereof; the amount of arginine or the pharmaceutically acceptable salt thereof in the composition being sufficient to maintain the pH of the composition within the range of 6 to 8 before parenteral administration.

In another general aspect, the pharmaceutical compositions according to the invention are provided for use in a method of treatment, control or prevention of a bacterial infection.

The details of one or more embodiments of the invention are set forth in the description below. Other features, objects and advantages of the invention will be apparent from the following description, including claims.

### DETAILED DESCRIPTION OF THE INVENTION

Reference will now be made to the exemplary embodiments, and specific language will be used herein to describe the same. It must be noted that, as used in this specification and the appended claims, the singular forms "a", "an", and "the" include plural referents unless the content clearly dictates otherwise.

The inventors have now surprisingly discovered that it is possible to treat bacterial infections using cefepime or a pharmaceutically acceptable salt thereof, tazobactam or a pharmaceutically acceptable salt thereof, and arginine or a pharmaceutically acceptable salt thereof. The compositions and methods disclosed herein also minimize or eliminate issues related to phlebitis.

The term "pharmaceutically acceptable salt" as used herein refers to one or more salts of a given compound which possesses the desired pharmacological activity of the free compound and which are neither biologically nor otherwise undesirable. In general, the "pharmaceutically acceptable salts" refer to and include those salts that are suitable for use in contact with the tissues of human and animals without undue toxicity, irritation, allergic response and the like, and are commensurate with a reasonable benefit/risk ratio. Pharmaceutically acceptable salts are well known in the art. For example, S. M. Berge, et al. (J. Pharmaceutical Sciences, 66: 1-19 (1977)) describes various pharmaceutically acceptable salts in details.

The term "infection" or "bacterial infection" as used herein refers to and includes presence of bacteria, in or on a subject, which, if its growth were inhibited, would result in a benefit to the subject. As such, the term "infection" in addition to referring to the presence of bacteria also refers to normal flora, which is not desirable. The term "infection" includes infections caused by bacteria.

The term "treat", "treating" or "treatment" as used herein refers to administering a medicament, including a pharmaceutical composition, or one or more active ingredients, for prophylactic and/or therapeutic purposes. The term "prophylactic treatment" refers to treating a subject who is not yet infected, but who is susceptible to, or otherwise at a risk of infection (preventing the bacterial infection). The term "therapeutic treatment" refers to administering treatment to a subject already suffering from infection. The terms "treat", "treating" or "treatment" as used herein also refer to administering compositions or one or more of active ingredients discussed herein, with or without additional active or inert ingredients, in order to: (i) reduce or eliminate either a bacterial infection or one or more symptoms of the bacterial infection, or (ii) retard the progression of a bacterial infection or of one or more symptoms of the bacterial infection, or (iii) reduce the severity of a bacterial infection or of one or more symptoms of the bacterial infection, or (iv) suppress the clinical manifestation of a bacterial infection, or (v) suppress the manifestation of adverse symptoms of the bacterial infection.

The term "pharmaceutically effective amount" or "therapeutically effective amount" or "effective amount" as used herein refers to an amount, which has a therapeutic effect or is the amount required to produce a therapeutic effect in a subject. For example, a therapeutically or pharmaceutically effective amount of an active ingredient or a pharmaceutical composition is the amount of the active ingredient or the pharmaceutical composition required to produce a desired therapeutic effect as may be judged by clinical trial results, model animal infection studies, and/or in vitro studies (e.g. in agar or broth media). The pharmaceutically effective amount depends on several factors, including but not limited to, the microorganism (e.g. bacteria) involved, characteristics of the subject (for example height, weight, sex, age and medical history), severity of the infection and the particular type of the antibacterial agent or active ingredient used. For prophylactic treatments, a therapeutically or prophylactically effective amount is that amount which would be effective in preventing a microbial (e.g. bacterial) infection. The active ingredients and/or pharmaceutical compositions according to the invention are used in amounts that are effective in providing the desired therapeutic effect or result.

The term "administration" or "administering" includes delivery of a composition, or one or more of active ingredients to a subject, including for example, by any appropriate methods, which serves to deliver the composition or the active ingredients to the site of the infection. The method of administration may vary depending on various factors, such as for example, the components of the pharmaceutical composition or the nature of the active and/or inert ingredients, the site of the potential or actual infection, the microorganism involved, severity of the infection and age and physical condition of the subject. Some examples of ways to administer a composition or active ingredients to a subject according to the invention include oral, intravenous, topical, intra-respiratory, intra-peritoneal, intra-muscular, parenteral, sublingual, transdermal, intranasal, aerosol, intra-ocular, intra-tracheal, intra-rectal, vaginal, gene gun, dermal patch, eye drop, ear drop or mouthwash. In case of a pharmaceutical composition comprising more than one ingredient (active or inert), one way to administering such composition is by admixing the ingredients (e.g. in the form of a suitable unit dosage form such as tablet, capsule, solution and powder) and then administering the dosage form. Alternatively, the ingredients may also be administered separately (simultaneously or one after the other) as long as these ingredients reach beneficial therapeutic levels such that the desired therapeutic effect is achieved.

The term "parenteral administration" refers to and includes a route of administration that does not involve gastrointestinal tract directly. Typical examples of parenteral route of administration includes intravenous (into a vein), intra-arterial (into an artery), intraosseous infusion (into the bone marrow), intra-muscular, intracerebral, intrathecal, subcutaneous administration. In general, the parenteral administration is performed by injecting or infusing the composition or the active ingredient(s) directly into a subject without direct involvement of the gastrointestinal tract.

The term "growth" as used herein refers to a growth of one or more microorganisms and includes reproduction or population expansion of the microorganism (e.g. bacteria). The term "growth" also includes maintenance of on-going metabolic processes of a microorganism (e.g. bacteria), including processes that keep the microorganism alive.

The term, "effectiveness" as used herein refers to the ability of a treatment or a composition or one or more active ingredients to produce a desired biological effect in a subject. For example, the term "antibacterial effectiveness" of a composition or an antibacterial agent refers to the ability of the composition or the antibacterial agent to treat or prevent the microbial (e.g. bacterial) infection in a subject.

The term "synergistic" or "synergy" as used herein refers to the interaction of two or more agents so that their combined effect is greater than their individual effects.

The term "antibacterial agent" as used herein refers to any substance, compound or a combination of substances or a combination of compounds capable of: (i) inhibiting, reducing or preventing growth of bacteria; (ii) inhibiting or reducing ability of a bacteria to produce infection in a subject; or (iii) inhibiting or reducing ability of bacteria to multiply or remain infective in the environment. The term "antibacterial agent" also refers to a compound capable of decreasing infectivity or virulence of bacteria.

The term "beta-lactam antibacterial agent" as used herein refers to compounds with antibacterial properties and containing a beta-lactam nucleus in their molecular structure.

The term "beta-lactamase" as used herein refers to any enzyme or protein or any other substance that breaks down a beta-lactam ring. The term "beta-lactamase" includes enzymes that are produced by bacteria and have the ability to hydrolyze the beta-lactam ring in a beta-lactam compound, either partially or completely.

The term "beta-lactamase inhibitor" as used herein refers to a compound capable of inhibiting activity of one or more beta-lactamase enzymes, either partially or completely.

The term "pharmaceutically inert ingredient" or "inert ingredient", "carrier" or "excipient" refers to a compound or material used to facilitate administration of a compound, including for example, to increase the solubility of the compound. Typical examples of solid carriers include, starch, lactose, di-calcium phosphate, sucrose, and kaolin and so on. Typical examples of liquid carriers include sterile water, saline, buffers, non-ionic surfactants, and edible oils such as oil, peanut and sesame oils and so on. In addition, various adjuvants commonly used in the art may be included. These and other such compounds are described in the literature, for example, in the Merck Index (Merck & Company, Rahway, N.J.). Considerations for inclusion of various components in pharmaceutical compositions are described, for example, in Gilman et al. (Eds.) (1990); Goodman and Gilman's: The Pharmacological Basis of Therapeutics, 8th Ed., Pergamon Press..

The term "subject" as used herein refers to a vertebrate or invertebrate, including a mammal. The term "subject" includes human, animal, a bird, a fish, or an amphibian. Typical examples of a "subject" includes humans, cats, dogs, horses, sheep, bovine cows, pigs, lambs, rats, mice and guinea pigs.

A person of skills in the art would appreciate that the compounds described herein can generally exist or used in various pharmaceutically acceptable forms including in the form of their pharmaceutically acceptable salts, pro-drugs, metabolites, esters, ethers, hydrates, polymorphs, solvates, complexes, enantiomers, adducts or such other pharmaceutically acceptable derivatives. A reference to the compound, therefore, is intended to include it's pharmaceutically acceptable salts, pro-drugs, metabolites, esters, ethers, hydrates, polymorphs, solvates, complexes, enantiomers, adducts or such other pharmaceutically acceptable derivative. For example, the terms "cefepime", "tazobactam", or "arginine" includes their pharmaceutically acceptable salts, pro-drugs, metabolites, esters, ethers, hydrates, polymorphs, solvates, complexes, enantiomers, adducts or such other pharmaceutically acceptable derivatives.

Each of cefepime or a pharmaceutically acceptable salt thereof, tazobactam or a pharmaceutically acceptable salt thereof, and arginine or a pharmaceutically acceptable salt thereof, is individually referred to as an "active ingredient" and collectively referred to as the "active ingredients". The terms "pharmaceutical compositions" or "composition" as used herein refer to and include the compositions according to the invention.

In one general aspect, there are provided pharmaceutical compositions suitable for parenteral administration, said compositions comprising: (a) 2 gram of cefepime or a pharmaceutically acceptable salt thereof, (b) 2 gram of tazobactam or a pharmaceutically acceptable salt thereof, and (c) arginine or a pharmaceutically acceptable salt thereof; the amount of arginine or the pharmaceutically acceptable salt thereof in the composition being sufficient to maintain the pH of the composition within the range of 6 to 8 before parenteral administration.

In some embodiments, tazobactam is present as tazobactam sodium. In some other embodiments, cefepime is present as cefepime hydrochloride. In some embodiments, arginine is present as arginine hydrochloride.

In some other embodiments, pH of the composition is within the range of about 6.5 to about 7.5 before parenteral administration.

In some embodiments, the compositions according to the invention consist of cefepime or a pharmaceutically acceptable salt thereof, tazobactam or a pharmaceutically acceptable salt thereof, and arginine or a pharmaceutically acceptable salt thereof, as the only active ingredients.

In some embodiments, arginine or a pharmaceutically acceptable salt thereof is present in the compositions according to the invention in an amount which is about 0.50 gram to about 0.90 gram, per gram of cefepime or a pharmaceutically acceptable salt thereof.

In some other embodiments, arginine or a pharmaceutically acceptable salt thereof is present in the compositions according to the invention in an amount which is about 0.70 gram to about 0.80 gram, per gram of cefepime or a pharmaceutically acceptable salt thereof.

In some embodiments, cefepime or the pharmaceutically acceptable salt thereof, tazobactam or the pharmaceutically acceptable salt thereof, and arginine or the pharmaceutically acceptable thereof, are present in the composition according to the invention in the following amounts:
(i) about 2 gram of cefepime or a pharmaceutically acceptable salt thereof, about 2 gram of tazobactam or a pharmaceutically acceptable salt thereof, and about 1.4 gram to about 1.6 gram of arginine or a pharmaceutically acceptable salt thereof;

The pharmaceutical compositions according to the invention may include one or more pharmaceutically acceptable carriers or excipients or inert ingredients. Typical examples of such carriers or excipients or inert ingredients include mannitol, lactose, starch, magnesium stearate, sodium saccharine, talcum, cellulose, sodium crosscarmellose, glucose, gelatin, sucrose, magnesium carbonate, wetting agents, emulsifying agents, solubilizing agents, buffering agents, lubricants, stabilizing agents and binding agents.

In some embodiments, the composition according to invention further comprises one or more buffering agent. Typical examples of buffering agents include aluminum hydroxide, aluminum hydroxide/magnesium carbonate co-precipitate, aluminum hydroxide/sodium bicarbonate co-precipitate, aluminium glycinate, aluminium magnesium hydroxide, aluminium phosphate, calcium acetate, calcium carbonate, calcium formate, calcium bicarbonate, calcium borate, calcium citrate, calcium gluconate, calcium glycerophosphate, calcium hydroxide, calcium chloride, calcium lactate, calcium phthalate, calcium phosphate, calcium succinate, calcium tartrate, calcium propionate, dibasic sodium phosphate, dipotassium hydrogen phosphate, dipotassium phosphate, disodium hydrogen phosphate, disodium succinate, dry aluminum hydroxide gel, magnesium acetate, magnesium aluminate, magnesium borate, magnesium bicarbonate, magnesium hydroxide, magnesium carbonate, magnesium citrate, magnesium gluconate, magnesium lactate, magnesium metasilicate aluminate, magnesium oxide, magnesium phthalate, magnesium phosphate, magnesium silicate, magnesium succinate, magnesium tartrate, potassium acetate, potassium carbonate, potassium bicarbonate, potassium borate, potassium citrate, potassium metaphosphate, potassium phthalate, potassium phosphate, potassium polyphosphate, potassium pyrophosphate, potassium succinate, potassium tartrate, sodium acetate, sodium bicarbonate, sodium borate, sodium carbonate, sodium citrate, sodium gluconate, sodium hydrogen phosphate, sodium hydroxide, sodium lactate, sodium phthalate, sodium phosphate, sodium polyphosphate, sodium pyrophosphate, sodium sesquicarbonate, sodium succinate, sodium tartrate, sodium tripolyphosphate, synthetic hydrotalcite, tetrapotassium pyrophosphate, tetrasodium pyrophosphate, tripotassium phosphate, trisodium phosphate, trometamol, trihydroxymethylaminomethane, an amino acid such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine or the optically active isomers thereof, or the racemic mixtures thereof, an acid salt of an amino acid, an alkali salt of an amino acid or mixtures thereof.

The pharmaceutical compositions according to the invention may be formulated into a variety of dosage forms, including solid, semi-solid, aerosol, and liquid dosage forms. Typical examples of dosage forms include tablets, capsules, powders, solutions, suspensions, suppositories, aerosols, granules, emulsions, syrups, elixirs and injectable preparations. If desired, the compositions according to the invention can also be prepared and packaged into a bulk form or into unit dosage forms.

The composition may also be formulated into a unit dosage form wherein the active ingredients (cefepime or a pharmaceutically acceptable salt thereof, tazobactam or a pharmaceutically acceptable salt thereof, and arginine or a pharmaceutically acceptable salt thereof) are present in admixture. Alternatively, the composition may also be formulated into a unit dosage form wherein cefepime or a pharmaceutically acceptable salt thereof, tazobactam or a pharmaceutically acceptable salt thereof, and arginine or a pharmaceutically acceptable salt thereof are present as separate components (for example, all three active ingredients in separate vials or dosage forms; any two active ingredients in one vial or a dosage form and the third active ingredient in a separate vial or a dosage form).

In some embodiments, the pharmaceutical compositions according to the invention are present in the form of a powder or a solution. In some other embodiments, the pharmaceutical compositions according to the invention are present in the form of a powder or a solution that can be reconstituted by addition of a compatible reconstitution diluent prior to administration.

In some embodiments, the pharmaceutical compositions according to the invention are present in the form of a powder that can be reconstituted by addition of a compatible reconstitution diluent prior to parenteral administration.

In some embodiments, the pharmaceutical compositions according to the invention are present in the form of a ready-to-use solution for parenteral administration.

In some embodiments, the pharmaceutical compositions according to the invention are present in the form of a solution that can be diluted further by addition of a compatible reconstitution diluent prior to parenteral administration.

In some embodiments, the pharmaceutical compositions according to the invention are present in the form of a powder as a unit dose contained in bottle or bag prior to parenteral administration. In some other embodiments, the pharmaceutical compositions according to the invention are present in the form of a solution as a unit dose contained in bottle or bag prior to parenteral administration.

In some other embodiments, the pharmaceutical compositions according to the invention are in the form of a frozen composition that can be diluted with a compatible reconstitution diluent prior to administration.

A wide variety of reconstitution diluents can be used. Typical examples of reconstitution diluent include water for injection, 0.9% sodium chloride solution, 5% dextrose solution and normal saline solution.

In another general aspect, the compositions according to the invention are for use in a method of treatment, control or prevention of bacterial infections. In some embodiments, the compositions according to the invention are for use in a method of treatment, control or prevention of bacterial infection in a subject, said method comprising administering to said subject an effective amount of a composition according to the invention. In some other embodiments, the compositions according to the invention are for use in a method of treatment, control or prevention of a bacterial infection in a subject, said method comprising parenteral administration of a pharmaceutical composition according to the invention.

In some other embodiments, there is provided a solution having a pH in the range within 6 to 8; said solution comprising 2 gram of cefepime or a pharmaceutically acceptable salt thereof, 2 gram tazobactam or a pharmaceutically acceptable salt thereof, and arginine or a pharmaceutically acceptable salt thereof; said arginine or the pharmaceutically acceptable salt being present in an amount sufficient to maintain the pH of the composition within the range between 6 to 8 before parenteral administration, for use in methods of treatment, control or prevention of bacterial infection in a subject, said methods comprising parenteral administration of said solution.

In some other embodiments, the solution has a pH within the range of about 6.5 to about 7.5.

In some other embodiments, cefepime or a pharmaceutically acceptable salt thereof, tazobactam or a pharmaceutically acceptable salt thereof, and arginine or a pharmaceutically acceptable thereof, are administered in the following amounts:
(i) about 2 gram of cefepime or a pharmaceutically acceptable salt thereof, about 2 gram of tazobactam or a pharmaceutically acceptable salt thereof, and about 1.4 gram to about 1.6 gram of arginine or a pharmaceutically acceptable salt thereof;

In other embodiments, the compositions according to the invention are administered by any appropriate method, which serves to deliver the composition or its constituents to the desired site. In case of administration of active ingredients, the active ingredients may also be administered by any appropriate method. The method of administration can vary depending on various factors, such as for example, the nature of the active ingredients or the composition, the site of the potential or actual infection, the microorganism involved, severity of infection, age and physical condition of the subject. Some examples of administration methods include intravenous, intraperitoneal, intramuscular, parenteral, intratracheal and intrarectal.

The compositions according to the invention comprise three active ingredients: cefepime or a pharmaceutically acceptable thereof, tazobactam or a pharmaceutically acceptable salt thereof, and arginine or a pharmaceutically acceptable salt thereof. A person of skills in the art would appreciate that these active ingredients can be formulated into various dosage forms wherein the active ingredients can be present either together (in mixture) or as separate components. When the active ingredients in the composition are formulated as a mixture, such composition can be delivered by administering such a mixture. The composition or dosage form wherein the active ingredients do not come as a mixture, but come as separate components, such composition/dosage form can be administered in several ways. In one possible way, the active ingredients can be mixed in the desired proportions and the mixture is then administered as required. Alternatively, the active ingredients can be separately administered in the appropriate amounts so as to achieve the same or equivalent therapeutic level or effect as would have been achieved by administration of the equivalent mixture. One or more inert ingredients or inactive ingredients can be also be used during formulation and/or administration, if desired.

In some embodiments, cefepime or a pharmaceutically acceptable salt thereof, tazobactam or a pharmaceutically acceptable salt, and arginine or a pharmaceutically acceptable salt thereof are administered simultaneously or one after the other. In some embodiments, the compositions or the active ingredients according to the invention are packed in the form of kit. The compositions or the active ingredients may be packed in one or more containers such as bottle, vial, syringes, boxes and bags. The kit may also include directions for use of the contents.

The compositions or the active ingredients according to the invention can be administered at varied time intervals depending upon the specific requirement or the desired therapeutic effect. In some embodiments, the compositions or the active ingredients according to the invention are administered one, two, three or four times a day. In some other embodiments, the compositions or the active ingredients according to the invention are administered every 4 hours, 6 hours, 8 hours, 12 hours or 24 hours.

In another general aspect, the compositions or the active ingredients according to the invention are used in prophylactic treatment of a subject, comprising administering to a subject at risk of infection caused by bacteria, a prophylactically effective amount a composition or the active ingredients according to the invention.

In general, the compositions or the active ingredients according to the invention are effective against infections caused by a wide variety of bacteria, including those exhibiting resistance to one or more of known antibacterial agents or compositions. Some examples of infections that can be treated, controlled or prevented using the compositions and methods according the invention include infections caused by bacteria belonging to genus *Escherichia, Staphylococcus, Streptococcus, Haemophilus, Klebsiella, Moraxella*, *Enterobacter, Proteus, Serratia, Pseudomonas, Acinetobacter, Citrobacter, Stenotrophomonas, Bacteroides, Prevotella, Fusobacterium, Clostridium.*

In general, the compositions or the active ingredients according to the invention are useful in treatment, control or prevention of several infections, including, for example, skin and soft tissue infections, febrile neutropenia, urinary tract infections, intraabdominal infections, respiratory tract infections, pneumonia (nosocomial), bacteremia, meningitis, diabetic foot infections, bone and joint infections, surgical site infections and Shigella dysentery.

### EXAMPLES

The following examples illustrate the embodiments of the invention that are presently best known. However, it is to be understood that the following are only exemplary or illustrative of the application of the principles of the present invention. Numerous modifications and alternative compositions, methods, and systems may be devised by those skilled in the art without departing from the spirit and scope of the present invention. The appended claims are intended to cover such modifications and arrangements. Thus, while the present invention has been described above with particularity, the following examples provide further detail in connection with what are presently deemed to be the most practical and preferred embodiments of the invention.

### Example 1

Antibacterial activity of cefepime combination with tazobactam against various bacterial strains, including those bacteria that are known to product one or more beta-lactamase, was investigated in quantitative drug diffusion assay performed as per CLSI recommendations (Clinical and Laboratory Standards Institute (CLSI), performance Standards for Antimicrobial Susceptibility Testing, 20th Informational Supplement, M 100 - S20, Volume 30, No. 1, 2010).

In a typical study, overnight grown bacterial cultures after appropriate dilution were seed inoculated in the molten, cooled agar media and plates poured. Antibacterial agents containing 6 mm diameter discs were placed on the top of the agar surface. Zone of inhibition based observation was performed after 16 to 18 hours of incubation at 35 ± 2°C in ambient air. The overall procedure was performed as per CLSI recommendations, and the results are presented in Table 1. These assays are routinely used in determining possibility of treating a particular infection using given antibacterial agent or a composition. In general, zone inhibition values in the sensitive (S) range indicate that the strain is susceptible to that antibacterial agent or composition. It is generally assumed that the antibacterial agent or the combination under consideration would not be effective in treating the infection, if the zone inhibition values are in the resistant (R) range. The CLSI based susceptibility assessment (that guides treatment decisions in an hospital/community setting) of these combinations suggested that, a combination of cefepime and tazobactam could convert the susceptibility profile of ESBL strains from 'Resistant' to 'Sensitive' suggesting favourable clinical utility of cefepime-tazobactam combination according to the invention.

| **Table 1.** Antibacterial activity of cefepime alone and in combination with tazobactam | | | |
|---|---|---|---|
| **Sr.** | **Bacterial strain** | Zone of Inhibition (mm) | |
| | | **Cefepime alone** | **Cefepime in combination with tazobactam (10 mcg)*** |
| 1. | *E. coli* M-138 | Nil (R) | 23 (S) |
| 2. | *E. coli* B-89 | 8 (R) | 20 (S) |
| 3. | *E. coli* B-123 | 8 (R) | 20 (S) |
| 4. | *E. coli* M50 | 7.5 (R) | 24 (S) |
| 5. | *E. coli* 7MP | 16(I) | 20.5 (S) |
| 6. | *E. coli* S-112 | 17(I) | 20.5 (S) |
| *(R): Resistant; (I): Intermediate; (S): Sensitive (Interpretation as per CLSI recommendations, 2010) * for possible treatment with Cefepime (0.5g)* +*Tazobactam (0.5g)* | | | |

### Example 2

A twelve day repeated dose study was undertaken to study effect arginine on the intravenous tolerability of the compositions comprising cefepime and tazobactam. Three groups of rats, each comprising 5 male Wistar rats (160-180 grams) were used in this study. First group of rats (Group I) was administered with cefepime for injection (400mg/kg dose). Second group of rats (Group II) was administered with a combination of cefepime for injection and tazobactam sodium (each of cefepime and tazobactam at a dose of 400 mg/kg in water for injection). Third group of rats (Group III) was administered with a combination of cefepime for injection and tazobactam sodium with additional arginine (each of cefepime and tazobactam at a dose of 400 mg/kg in 5 ml water for injection containing 4 mg/ml of arginine). In a typical procedure, a new vial was opened every day, added required quantity of arginine diluent and pH was recorded. Further it was diluted with 0.9% Saline to make final concentration of 100 mg/mL and pH was recorded. The failure to administer dose due to phlebitis was counted in each case, and the results are presented in Table 2. The failed dosing attempt due to loss of tail vein integrity was counted in case of rats.

As can be seen from the results given in Table 2, the percentage of failed dosing attempts was about 13.1% for administration of cefepime for injection, which has a pH within the range of about 4.1 to 4.2. The percentage of failed dosing attempts was even higher (about 15.1%) for administration of a solution containing cefepime for injection and tazobactam sodium (pH within the range of about 3.8 to 4.1). Surprisingly, the percentage of failed dosing attempts significantly lowered to about 3.1% when the same composition containing cefepime for injection and tazobactam sodium was administered with the addition of arginine and adjusting the pH of the solution before administration to a range within about 6 to about 7.4. The study confirms less frequency of failed dosing attempts following addition of arginine into the composition and also adjustment of the pH operating range (less occurrence of phlebitis).

### Example 3

Several compositions containing the active ingredients in the disclosed amounts were prepared in the form of a powder and solutions. Some compositions were also prepared in the form of solutions having pH within the disclosed range.

| **Table 2.** Effect of arginine on intravenous tolerability of a composition comprising cefepime and tazobactam | | | | | | | | | | | | | | |
|---|---|---|---|---|---|---|---|---|---|---|---|---|---|---|
| Group of rats | pH of the solution before administration | ***Dosing Day*** | | | | | | | | | | | | Number of failed dosing attempts (%) |
| | | ***1*** | **2** | **3** | **4** | ***5*** | **6** | **7** | **8** | **9** | ***10*** | ***11*** | ***12*** | |
| **Group (I)** *(cefepime for injection)* | 4.0 to 4.2 | 0 | 0 | 0 | 1 | 1 | 2 | 3 | 3 | 3 | 4 | 4 | 4 | 25 (13.1) |
| **Group (II)** *(cefepime for injection* + *tazobactam sodium)* | 3.8 to 4.1 | 0 | 0 | 0 | 1 | 0 | 1 | 4 | 4 | 2 | 5 | 6 | 6 | 29 (15.1) |
| **Group (III)** *(cefepime for injection* + *tazobactam sodium* + *added arginine)* | 6.0 to 7.4 | 0 | 0 | 1 | 1 | 1 | 0 | 0 | 1 | 0 | 1 | 0 | 1 | 6(3.1) |

## Claims

1. A pharmaceutical composition suitable for parenteral administration, said composition comprising: (a) 2 gram of cefepime or a pharmaceutically acceptable salt thereof, (b) 2 gram of tazobactam or a pharmaceutically acceptable salt thereof, and (c) arginine or a pharmaceutically acceptable salt thereof; the amount of arginine or a pharmaceutically acceptable salt thereof in the composition being sufficient to maintain the pH of the composition within the range of 6 to 8 before parenteral administration.

2. The pharmaceutical composition according to Claim 1, wherein the pH of the composition is within the range of 6.5 to 7.5.

3. The pharmaceutical composition according to Claim 1 or 2, wherein arginine or a pharmaceutically acceptable salt thereof is present in the composition in an amount which is 0.50 gram to 0.90 gram, per gram of cefepime or a pharmaceutically acceptable salt thereof.

4. The pharmaceutical composition according to Claim 1 or 2, wherein arginine or a pharmaceutically acceptable salt thereof is present in the composition in an amount which is 0.70 gram to 0.80 gram, per gram of cefepime or a pharmaceutically acceptable salt thereof.

5. The pharmaceutical composition according to Claim 1 or 2, comprising 2 gram of cefepime or a pharmaceutically acceptable salt thereof, 2 gram of tazobactam or a pharmaceutically acceptable salt thereof, and 1.4 gram to 1.6 gram of arginine or a pharmaceutically acceptable salt thereof.

6. The pharmaceutical composition according to any one of the Claims 1 to 5, said composition in the form of a powder that can be reconstituted by addition of a compatible reconstitution diluent prior to parenteral administration

7. The pharmaceutical composition according to any one of the Claims 1 to 5, said composition in the form of a solution ready to use for parenteral administration.

8. The pharmaceutical composition according to any one of the Claim 1 to 5, said composition in the form of a solution that can be diluted further by addition of a compatible reconstitution diluent prior to parenteral administration

9. The pharmaceutical composition according to any one of the Claims 1 to 5, wherein the composition in the form of a powder, and is a unit dose contained in bottle or bag prior to parenteral administration.

10. The pharmaceutical composition according to any one of the Claims 1 to 5, wherein the composition in the form of a solution, and is a unit dose contained in bottle or bag prior to parenteral administration.

11. The pharmaceutical composition according to Claim 1, wherein cefepime or a pharmaceutically acceptable salt thereof, tazobactam or a pharmaceutically acceptable salt thereof, and arginine or a pharmaceutically acceptable salt thereof are present in the composition as admixture or as separate components.

12. Pharmaceutical composition according to any one of the Claims 1 to 11, for use in a method of treatment, control or prevention of a bacterial infection.

## Patentansprüche

1. Pharmazeutische Zusammensetzung zur parenteralen Verabreichung geeignet, wobei die Zusammensetzung umfasst:
(a) 2 Gramm Cefepim oder eines pharmazeutisch verwendbaren Salzes davon,
(b) 2 Gramm Tazobactam oder eines pharmazeutisch verwendbaren Salzes davon und
(c) Arginin oder ein pharmazeutisch verwendbares Salz davon, wobei die Menge von Arginin oder eines pharmazeutisch verwendbaren Salzes davon in der Zusammensetzung ausreichend ist, um den pH-Wert der Zusammensetzung vor einer parenteralen Verabreichung im Bereich von 6 bis 8 beizubehalten.

2. Pharmazeutische Zusammensetzung nach Anspruch 1, wobei der pH-Wert der Zusammensetzung im Bereich von 6,5 bis 7,5 liegt.

3. Pharmazeutische Zusammensetzung nach Anspruch 1 oder 2, wobei Arginin oder ein pharmazeutisch verwendbares Salz davon in der Zusammensetzung in einer Menge vorhanden ist, die bei 0,50 Gramm bis 0,90 Gramm pro Gramm von Cefepim oder eines pharmazeutisch verwendbaren Salzes davon liegt.

4. Pharmazeutische Zusammensetzung nach Anspruch 1 oder 2, wobei Arginin oder ein pharmazeutisch verwendbares Salz davon in der Zusammensetzung in einer Menge vorhanden ist, die bei 0,70 Gramm bis 0,80 Gramm pro Gramm von Cefepim oder eines pharmazeutisch verwendbaren Salzes davon liegt.

5. Pharmazeutische Zusammensetzung nach Anspruch 1 oder 2, umfassend 2 Gramm Cefepim oder eines pharmazeutisch verwendbaren Salzes davon, 2 Gramm Tazobactam oder eines pharmazeutisch verwendbaren Salzes davon und 1,4 Gramm bis 1,6 Gramm Arginin oder eines pharmazeutisch verwendbaren Salzes davon.

6. Pharmazeutische Zusammensetzung nach einem der Ansprüche 1 bis 5, die Zusammensetzung in Form eines Pulvers, die durch das Hinzufügen eines kompatiblen Rekonstitutionsverdünners vor einer parenteralen Verabreichung wiederhergestellt werden kann.

7. Pharmazeutische Zusammensetzung nach einem der Ansprüche 1 bis 5, die Zusammensetzung in Form einer zur parenteralen Verabreichung gebrauchsfertigen Lösung.

8. Pharmazeutische Zusammensetzung nach einem der Ansprüche 1 bis 5, die Zusammensetzung in Form einer Lösung, die durch das Hinzufügen eines kompatiblen Rekonstitutionsverdünners vor einer parenteralen Verabreichung weiter verdünnt werden kann.

9. Pharmazeutische Zusammensetzung nach einem der Ansprüche 1 bis 5, wobei die Zusammensetzung in Form eines Pulvers und vor einer parenteralen Verabreichung eine in einer Flasche oder einem Beutel enthaltene Einheitsdosis ist.

10. Pharmazeutische Zusammensetzung nach einem der Ansprüche 1 bis 5, wobei die Zusammensetzung in Form einer Lösung und vor einer parenteralen Verabreichung eine in einer Flasche oder einem Beutel enthaltene Einheitsdosis ist.

11. Pharmazeutische Zusammensetzung nach Anspruch 1, wobei Cefepim oder ein pharmazeutisch verwendbares Salz davon, Tazobactam oder ein pharmazeutisch verwendbares Salz davon und Arginin oder ein pharmazeutisch verwendbares Salz davon in der Zusammensetzung als Zumischung oder als getrennte Inhaltsstoffe vorhanden sind.

12. Pharmazeutische Zusammensetzung nach einem der Ansprüche 1 bis 11, zur Verwendung bei einem Behandlungsverfahren, Kontrolle oder Verhinderung einer bakteriellen Infektion.

## Revendications

1. Composition pharmaceutique appropriée pour une administration parentérale, ladite composition comprenant : (a) 2 grammes de céfépime ou d'un sel pharmaceutiquement acceptable de celui-ci, (b) 2 grammes de tazobactame ou d'un sel pharmaceutiquement acceptable de celui-ci, et (c) de l'arginine ou un sel pharmaceutiquement acceptable de celle-ci ; la quantité d'arginine ou d'un sel pharmaceutiquement acceptable de celle-ci dans la composition étant suffisante pour maintenir le pH de la composition dans la plage de 6 à 8 avant une administration parentérale.

2. Composition pharmaceutique selon la revendication 1, dans laquelle le pH de la composition est dans la plage de 6,5 à 7,5.

3. Composition pharmaceutique selon la revendication 1 ou 2, dans laquelle l'arginine ou un sel pharmaceutiquement acceptable de celle-ci est présent(e) dans la composition en une quantité qui est de 0,50 gramme à 0,90 gramme, par gramme de céfépime ou d'un sel pharmaceutiquement acceptable de celui-ci.

4. Composition pharmaceutique selon la revendication 1 ou 2, dans laquelle l'arginine ou un sel pharmaceutiquement acceptable de celle-ci est présent dans la composition en une quantité qui est de 0,70 gramme à 0,80 gramme, par gramme de céfépime ou d'un sel pharmaceutiquement acceptable de celui-ci.

5. Composition pharmaceutique selon la revendication 1 ou 2, comprenant 2 grammes de céfépime ou d'un sel pharmaceutiquement acceptable de celui-ci, 2 grammes de tazobactame ou d'un sel pharmaceutiquement acceptable de celui-ci, et 1,4 gramme à 1,6 gramme d'arginine ou d'un sel pharmaceutiquement acceptable de celle-ci.

6. Composition pharmaceutique selon l'une quelconque des revendications 1 à 5, ladite composition étant sous la forme d'une poudre qui peut être reconstituée par addition d'un diluant de reconstitution compatible avant une administration parentérale.

7. Composition pharmaceutique selon l'une quelconque des revendications 1 à 5, ladite composition étant sous la forme d'une solution prête à l'emploi pour une administration parentérale.

8. Composition pharmaceutique selon l'une quelconque des revendications 1 à 5, ladite composition étant sous la forme d'une solution pouvant être diluée davantage par addition d'un diluant de reconstitution compatible avant une administration parentérale

9. Composition pharmaceutique selon l'une quelconque des revendications 1 à 5, dans laquelle la composition est sous la forme d'une poudre, et est une dose unitaire contenue dans une bouteille ou une poche avant une administration parentérale.

10. Composition pharmaceutique selon l'une quelconque des revendications 1 à 5, dans laquelle la composition est sous la forme d'une solution, et est une dose unitaire contenue dans une bouteille ou une poche avant une administration parentérale.

11. Composition pharmaceutique selon la revendication 1, dans laquelle du céfépime ou un sel pharmaceutiquement acceptable de celui-ci, du tazobactame ou un sel pharmaceutiquement acceptable de celui-ci, et de l'arginine ou un sel pharmaceutiquement acceptable de celle-ci sont présents dans la composition sous la forme d'un mélange ou de composants séparés.

12. Composition pharmaceutique selon l'une quelconque des revendications 1 à 11, à utiliser dans un procédé de traitement, de commande ou de prévention d'une infection bactérienne.
